# EUROPEAN PATENT APPLICATION

(11) **EP 4 205 706 A1**
(43) Date of publication of application: **05.07.2023**
(21) Application number: 21218396.6
(22) Date of filing: 30.12.2021
(51) Int. Cl.: A61F 2/38, A61F 2/30

(54) **A FLEXIBLE MENISCUS PROSTHESIS**

(71) Applicant: Atro Medical B.V., 6534 AT Nijmegen (NL)
(72) Inventor: van Tienen, Tony George, 6534 AT Nijmegen (NL); van der Veen, Albert Jan, 6534 AT Nijmegen (NL); van Minnen, Branco Siebren, 6534 AT Nijmegen (NL); Hunik, Jan Hendrik, 6534 AT Nijmegen (NL)
(74) Representative: Nederlandsch Octrooibureau

(57) **Abstract**

An arc-shaped meniscus prosthesis comprises a first end (201) having a first fastening part (203) for securing the first end to a bone surface and a second end (202) having a second fastening part (204) for securing the second end to a bone surface. A curved intermediate section (203) connects the first end and the second end. The curved intermediate section is made of a single first biocompatible, non-resorbable material that has a tensile modulus of at most 160 MPa. The first material is isotropic with regard to the tensile modulus and the intermediate section is a monolithic piece of the first material.

## Description

### FIELD OF THE INVENTION

The invention relates to a flexible meniscus prosthesis. The invention further relates to a method of manufacturing a meniscus prosthesis.

### BACKGROUND OF THE INVENTION

The meniscus is a C-shaped ring in the knee joint that shares the vertical load of the femoral condyle with the cartilage of the tibia, thus enlarging the loaded area of the cartilage, and also acts as a sliding bearing to lubricate the joint. The native meniscus shows an anisotropic behaviour to withstand the hoop stress and compression between the femur and tibia bone.

Known attempts to create a prosthesis to replace the meniscus follow this anisotropic design with flexible materials with comparable stiffness in compression but reinforced with fibers or stiff rings to approach the stiff elastic modulus of the meniscus in circumferential direction.

Fig. 1 shows a meniscus prosthesis as disclosed in European patent EP3116448, with a core 2 made of a first biocompatible, non-resorbable material having a first tensile modulus, and a cushioning material 1 that surrounds the intermediate section of the core, the cushioning material 1 being made of a second biocompatible, non-resorbable material having a second tensile modulus, which is lower than a tensile modulus of the first material. The meniscus prosthesis further comprises fixation parts 2A and 2B with first and second through holes 3A and 3B.

US10918486 B2 discloses a meniscus prosthesis incorporating a wire/fiber structure that extends from one end to the other end of the prosthesis. These ends are secured in the bone. The prosthesis body woven structure is molded into a more flexible material which contacts the cartilage and compresses easily. This leads to a high stiffness in circumferential direction and lower stiffness in all other directions.

### SUMMARY OF THE INVENTION

It would be advantageous to provide a prosthesis that is more durable and/or more comfortable for the patient.

According to a first aspect of the invention, an arc-shaped meniscus prosthesis is provided, comprising
a first end having a first through-hole configured to receive a connector for securing the first end to a bone surface;
a second end having a second through-hole configured to receive a connector for securing the second end to a bone surface;
a curved intermediate section connecting the first end and the second end,
wherein the curved intermediate section is made of a single first biocompatible, non-resorbable material or composition that has a tensile modulus of at most 160 MPa as determined by ISO 527-1.

The material or composition of the curved intermediate section provides a relatively high flexibility in the circumferential direction of the curved intermediate section. This in turn provides an improved performance of the prosthesis. Next to deformation during compression, it may allow adaptation to the changing geometries of the joint surfaces during movement and/or loading of the joint by means of elastic deformation in the circumferential direction, i.e. reversible and temporary deformation. Surprisingly, this may help to make the prosthesis more durable, by virtue of lower stresses on the prosthesis. Moreover, this prosthesis may improve patient comfort by virtue of more evenly distributed loads, less risk of limitation in range of motion, and/or reduced chance of impingement.

The intermediate section may be a monolithic piece of material. This helps to simplify manufacturing and is made possible because the middle section is isotropic with respect to the modulus of elasticity, so that no additional reinforcement of the middle section may be needed. Similarly, the intermediate section may consist of a single component.

According to another aspect of the invention, a method of manufacturing an arc-shaped meniscus prosthesis is provided, comprising
molding at least part of a first end of the arc-shaped meniscus prosthesis, the at least part of the first end having a first fastening part for securing the first end to a bone surface;
molding at least part of a second end of the arc-shaped meniscus prosthesis, the at least part of the second end having a second fastening part for securing the second end to a bone surface; and
molding a curved intermediate section connecting the first end and the second end,
wherein the curved intermediate section is made of a first biocompatible, non-resorbable material that has a tensile modulus of at most 160 MPa as determined by ISO 527-1.

The person skilled in the art will understand that the features described above may be combined in any way deemed useful. Moreover, modifications and variations described in respect of the system may likewise be applied to the method and to the computer program product, and modifications and variations described in respect of the method may likewise be applied to the system and to the computer program product.

### BRIEF DESCRIPTION OF THE DRAWINGS

In the following, aspects of the invention will be elucidated by means of examples, with reference to the drawings. The drawings are diagrammatic and may not be drawn to scale. Throughout the drawings, similar items may be marked with the same reference numerals.
Fig. 1 shows a prior art meniscus prosthesis.
Fig. 2 shows a meniscus prosthesis in accordance with an embodiment of the present invention.
Fig. 3 shows a cross section of the meniscus prosthesis of Fig. 2.
Fig. 4 shows an end portion of a meniscus prosthesis in accordance with an embodiment of the present invention.
Fig. 5 shows examples of different shapes of a connecting surface between an end portion and an intermediate section, as follows.
Fig. 5A shows a planar interface.
Fig. 5B shows a donut shaped interface.
Fig. 5C shows an adjusted donut shape surface.
Fig. 5D shows an interface having the shape of a short block.
Fig. 5E shows an interface having the shape of a long block.
Fig. 5F shows an interface with a short wedge.
Fig. 5G shows an interface with a long wedge.
Fig. 6 shows an example of fixation of the end portions with screws.
Fig. 7 shows an example of fixation of the end portions with tape.
Fig. 8A shows a tape.
Fig. 8B shows a tape with a knot.
Fig. 8C shows a tape with a loophole.
Fig. 8D shows a tape with tapered ends.
Fig. 8E shows a tape in style of a brake cable.
Fig. 8F shows a tape with a knot on one end and a tapered end.
Figs. 9A through 9E show the end portion with a tape in various positions.
Fig. 10A shows an end portion with an oval through hole.
Fig. 10B shows an end portion with a rectangular through hole.

### DETAILED DESCRIPTION OF EMBODIMENTS

Certain exemplary embodiments will be described in greater detail, with reference to the accompanying drawings.

The matters disclosed in the description, such as detailed construction and elements, are provided to assist in a comprehensive understanding of the exemplary embodiments. Accordingly, it is apparent that the exemplary embodiments can be carried out without those specifically defined matters. Also, well-known operations or structures are not described in detail, since they would obscure the description with unnecessary detail.

The meniscus is a C-shaped ring in the knee joint which redistributes the loads from the femoral condyle to the tibia, thus lowers the peak pressures on the tibial cartilage, enhances the bearing function of the joint, and increases the in-plane stability of the joint. This meniscus is soft in compression but very stiff in circumferential direction. It is very difficult to copy these mechanical properties with an artificial structure. Most attempts make use of flexible materials with comparable tensile modulus in compression but reinforced with fibers or stiff rings to mimic the stiff elastic modulus in circumferential direction. For example, EP3116448, discloses a stiff ring to achieve such tensile modulus and a flexible polymer which covers this stiff ring with a flexible polymer.

With the currently available materials and manufacturing methods it is a major challenge to further improve the comfort and/or durability of a meniscus prosthesis. overcome such problems. The innovative idea to opt for a meniscus prosthesis made of a highly flexible material may seem counter-intuitive, but it may be advantageous that it allows the implant to be pushed more out of the joint than the natural meniscus and it can share the load together with the articular cartilage. Although less of the force is carried by the implant and more by the underlying cartilage, this load on the cartilage is still less than without a meniscus implant and as long as the meniscus implant stays in close contact with the femoral condyle and the tibia plateau, it still acts as a bearing that contains the synovial fluid lubricant within the joint surfaces.

An exemplary arc-shaped meniscus prosthesis, in accordance with the present invention, comprises a first end having a first fixation part for securing the first end to a bone surface, a second end having a second fixation part for securing the second end to a bone surface, and a curved intermediate section connecting the first end and the second end. The curved intermediate section is made of a single first biocompatible, non-resorbable material that has a tensile modulus of at most 160 MPa as determined by ISO 527-1. It is noted that the material may be a composition comprising several ingredients, for example the material may be a homogeneous composition of several component materials. The intermediate section made of the first material may be more isotropic with regard to the tensile modulus than the native meniscus. For example, the intermediate section may be a monolithic molded piece of the first material. This may help to ensure the isotropy and elasticity. Surprisingly, it was found that this may improve the durability of the intermediate section. In certain embodiments, the first material is isotropic with regard to the tensile modulus.

Preferably, the first material has a tensile modulus between 0.1 MPa and 160 MPa measured according to ISO 527-1. This was found to provide comfort and durability. More preferably, the first material has a tensile modulus between 5 MPa and 100 MPa measured according to ISO 527-1, for better comfort and durability. Even more preferably, the first material has a tensile modulus between 5 MPa and 15 MPa.

The tensile modulus of the components of the meniscus prosthesis are preferably selected so that the meniscus prosthesis as a whole is stiff enough in the horizontal plane, the plane of the tibia surface, which corresponds generally to a plane intersecting the first end portion, the second end portion, and the intermediate section halfway between the first end portion and the second end portion, to, when secured to the bone surface at the first end and at the second end, prevent dislocation of the intermediate section. For example, a dislocation comparable to a 'buckethandle tear' dislocation in the native meniscus can be avoided in this way. In this way the prosthesis does not need any attachment to the peripheral capsule or tibia bone (or femur bone), apart from the fastening parts at the ends.

The first end can comprise a second biocompatible, non-resorbable material that has a tensile modulus of at least 100 MPa as determined by ISO 527-1. Similarly, the second end can comprise a third biocompatible, non-resorbable material that has a tensile modulus of at least 100 MPa as determined by ISO 527-1. Herein, the second material and the third material can be the same material or different materials. The higher tensile modulus of the first and second ends makes the ends stiffer and more wear resistant and moreover helps to distribute the forces exerted on the respective fixation parts. Preferably, the second material and the third material each have a tensile modulus between 100 MPa and 3500 MPa measured according to ISO 527-1. More preferably, the second material and the third material each have a tensile modulus between 100 MPa and 1000 MPa, even more preferably between 101 MPa and 250 MPa, measured according to ISO-527-1. It helps if the tensile modulus of the end parts is not too high, because its softness provides comfort and may improve the durability of the prosthesis.

The first end may be made entirely of the second material and the second end may be made entirely of the third material. This provides good strength near the fixation parts.

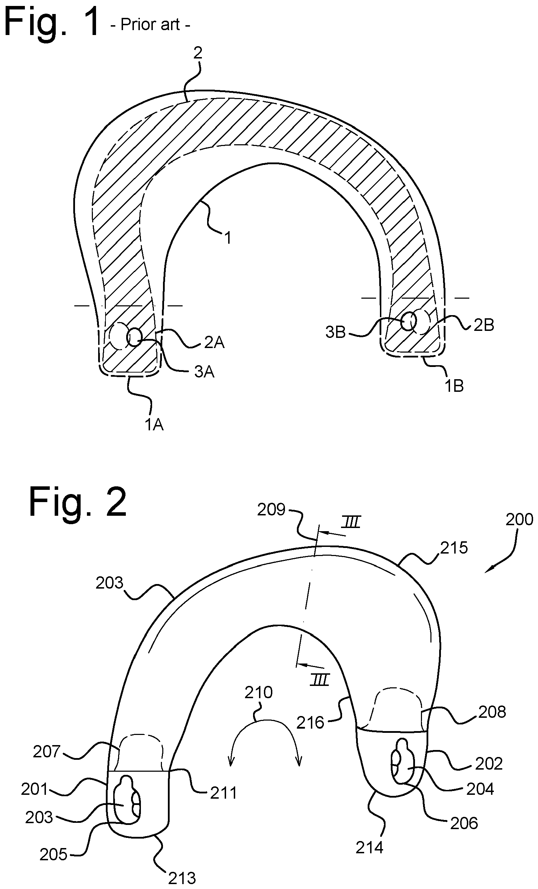

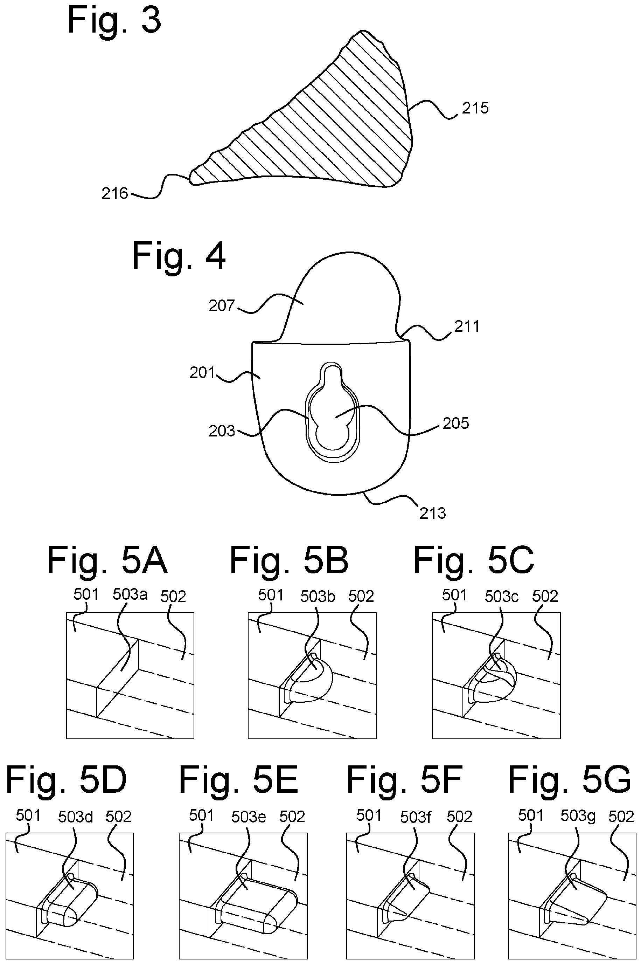

The end parts are connected to the intermediate part. The geometry of these ends and the way these ends are connected to the intermediate section may differ. The first end may comprise a first protrusion, wherein the first protrusion engages with a corresponding recess in the middle section, as illustrated in Fig 2 and Fig. 5). Similarly, the second end may comprise a second protrusion, wherein the second protrusion engages with a corresponding recess in the middle section. The first protrusion may be made of the second material, and the second protrusion may be made of the third material. The ends' protrusions may have different geometries or no protrusion at all (Fig 5). For example, the first end may be a monolithic or single-component piece of material comprising the first protrusion, and the second end may be a monolithic or single-component piece of material comprising the second protrusion.

Alternatively, the first end and/or the second end comprises a stiffer core made of the second material and a cushioning material that covers the core. For example, the cushioning material may be the first material. Moreover, the stiffer core of each end may be embedded in the material of the intermediate section. This provides additional softness at the ends.

For example, a minimum transverse cross-sectional area of the curved intermediate section may be at least 8 mm². This would help to provide the desired support and comfort of the meniscus prosthesis.

In certain embodiments, preferably a width of the curved section, measured from the inner circumference 216 to the outer circumference 215, is at most 3 cm, preferably at most 2 cm. In particular the prosthesis leaves at least part of the tibia plateau surface uncovered. This would help to provide the desired support and comfort of the meniscus prosthesis.

The first material may comprise or be a thermoplastic material, preferably a polyurethane, more preferably a polycarbonate urethane. Similarly, the second material may comprise or be a thermoplastic material, preferably a polyurethane, more preferably a polycarbonate urethane. In an alternative implementation, the first material may be a thermoplastic material, whereas the second and third material may be of another kind of material, such as a ceramic.

Preferably, the first fixation part comprises a first through-hole configured to receive a connector for securing the first end to a bone surface. Likewise, preferably, the second fixation part comprises a second through-hole configured to receive a connector for securing the second end to a bone surface.

The meniscus prosthesis may be manufactured by means of molding. For example, a method of manufacturing an arc-shaped meniscus prosthesis can comprise molding at least part of a first end of the arc-shaped meniscus prosthesis, the at least part of the first end having a first fixation part for securing the first end to a bone surface, and molding at least part of a second end of the arc-shaped meniscus prosthesis, the at least part of the second end having a second fixation part for securing the second end to a bone surface. Next, the method may proceed by molding a curved intermediate section connecting the first end and the second end, wherein the curved intermediate section is made of a first biocompatible, non-resorbable material that has a tensile modulus of at most 160 MPa as determined by ISO 527-1. For example, the molding of the curved intermediate section is performed in such a way that the material of the curved intermediate section touches a part of the surface of the first end and a part of the surface of the second end, for example it touches the surface of a stub. During the molding procedure these different materials will attach to each other at the touching surface.

For example, the meniscus prosthesis disclosed herein may have a geometry that corresponds to the geometry of the anatomic meniscus.

Moreover, the meniscus prosthesis may be made mostly or entirely of a material that has a meniscus-like elastic modulus in compression, but with a much lower elastic modulus in circumferential direction.

The middle section and/or end sections may be non-resorbable. This helps to make the implant more durable, so that it can be optimally used as a permanent prosthesis. The material used for the middle section and/or end sections may contain self-assembling monolayer end groups. This helps to provide a film on the surface that improves comfort.

An advantage of the meniscus prosthesis disclosed herein is that chances on failure will decrease while the implant is allowed to be further pushed out of the joint, without exceeding the ultimate tensile strength of the material, while remaining in tight contact with the femur and tibia to fill the wedge shape joint space and fulfill its bearing function. In case of high load, the load may be (partially) shifted from load on the implant to load on the cartilage.

In certain embodiments, the meniscus prosthesis may have an end section that is stiffer than the larger portion of the meniscus prosthesis. This end section may be made of a stiffer material, in order to strengthen the connection to the bone of the patient. Such an end section may be present at both ends of the C-shape. For example, the end section may comprise a connection means, such as a hole. The flexible section in between the two end sections may be referred to as a middle section. Furthermore, the increased flexibility of the middle section allows this part of the implant to continuously follow the changing shape of the condyle during flexion.

In certain embodiments, the end section may have a small stub that extends into the flexible material of the middle section. This increases the tear strength in shear force conditions, so that the durability of the implant in the joint may be increased. This stub should only protrude to a limited extent into the middle section of the implant, otherwise it will negatively affect the desired high elasticity of the middle section, i.e. the implant will become too stiff in-plane of the tibial plateau and will not be able to adjust itself the shape of the condyle.

The meniscus prosthesis may be connected to the bone with 2 screws, one for each end section.

Alternatively, the meniscus prosthesis may be connected to the bone with two sutures, tapes, ropes or wires. The fixation of an implant to bone may be critical, especially when it is desirable that the implant has some freedom to move in the joint or other location in the body where the implant is placed. When the fixation is rigid, as with screws, motion may be realized by deformation of the implant alone, which can lead to high loads, which may in turn lead to an increased risk of (fatigue) failure. Therefore, preferably the fixation (be it with screws or tapes or other fixation manner) allows the prosthesis to rotate around the fixation point, and preferably allows some translational freedom. When the fixation method also allows the implant to move, as with a tape configuration, the chance of failure may reduce. Nevertheless, preferably the fixation part (between the flexible body and the fixation structure (e.g. sutures, tapes, ropes or screws) may be made of a material that is stiffer than the middle section, to resist wear against the fixation structure (tape/screw) and creep. When the same flexible material is used for the fixation part as for the body, i.e. a 1-component implant, much or all of the loading may concentrate on the location around the fixation hole: the strength, due to a reduced cross section, may be lower at the hole and the risk of local large strains of and wear of the fixation eyelet (hole) will increase. With the introduction of the stiffer material between the fixation (tape/screw) and the flexible body (middle section), the transition of the stiffness of the different materials (tape/screw→ stiff polymer→ flexible polymer) is more gradual and reduces the chances of breakage of the implant due to fatigue or wear. However, it will be understood that although the stiffer fixation part may have such advantages, in certain embodiments the same flexible material is used for the fixation part as for the body.

The connection between the flexible and stiff material of the implant needs to be able to withstand the applied load. A connection between two flat surfaces (a surface of the flexible section and a surface of the stiff section) may be easily created and may be strong enough in pure tension. However, the direction of the force in the knee joint in that area is not perfectly longitudinal but can come along with shear forces in all different directions. Furthermore, the transition from rigid to flexible introduces a stress concentration. Therefore, there may be a risk that the implant breaks in the flexible material next to the sharp transition of the flexible to the stiff material. Such a crack may progress along the interface plane between the flexible material and the stiff material. With an extension of the stiff material protruding into the flexible body material, this extension realizes a gradual transition of forces along the longitudinal axis of the implant horn. Alternatively the flexible body material could be made to protrude into the more stiff material.

Surprisingly, it was found that breakage may be prevented by providing a more flexible implant body compared to the prior art implant body. The implant has the function to take over the function of the resected native meniscus: In particular, to share the vertical load from the femoral condyle between the meniscus and the part of tibial cartilage which is in direct contact with the condyle, and to avoid the situation that the tibial cartilage carries all the load after the meniscus is surgically removed. But all knees (or joints) have different forms and sizes. The more flexible the implant, the more it can adapt to different knees by means of reversible deformation and permanent creep. Furthermore, the meniscus acts as a sliding bearing with the synovial fluid as lubricant. When the meniscus is removed, the lubricant is no longer able to build up a pressurized fluid cushion at its contact area. The meniscus prostheses as disclosed herein was found to be highly suitable to take over this function from the meniscus.

In certain embodiments, the implant has a wedge shape, in which the implant is thicker at the outer circumference than at the inner circumference. In certain embodiments, the inside of the arc-shape has no material and allows direct femur to tibia contact. The wedge shape may facilitate that the implant may be (partially) pushed out of the joint during loading. However, this effect may also be apparent if the implant has a different shape.

In the following, certain features will be explained in greater detail with reference to the drawings. However, it will be understood these details are intended as an aid to understand the invention rather than to limit the invention.

Fig. 2 shows a meniscus prosthesis according an embodiment of the present invention. As shown in Fig. 2, the prosthesis body 200 comprises the intermediate section 203, which constitutes the main portion of the arc-shaped meniscus prosthesis body, and two end portions 201 and 202. The end portions 101 and 102 are configured to be secured to the bone within the joint.

Fig. 3 shows an example of a cross section of the intermediate section. As illustrated, the cross section has a wedge shape tapering towards the inner circumference 216 and with the largest thickness at the outer circumference 215 of the C shape. However, other design of the cross section may be contemplated.

Fig. 4 shows the first end portion 201 in greater detail. The end portions 201, 202 contain fastening parts 203, 204. The fastening parts 203, 204 form at least a portion of the two end portions 201, 202. The intermediate section 203 is made of a first material, in particular a first biocompatible non-resorbable material having a first tensile modulus. In the illustrated embodiment, the fastening parts 203, 204 of the end portions 201, 202 each comprise a through hole 205, 206. The through holes 205, 206 are configured to receive a connector to fasten the meniscus prosthesis onto the bone (e.g. the tibia). These through holes 205, 206 may be made a bit elongated or oval, to allow the meniscus prosthesis a bit of movement with respect to the bone. In alternative embodiments the through hole 205, 206 may be omitted and the fastening parts 203, 204 may be implemented, for example, by means of a piece of tape or suture or a pin extending from each end portion 201, 202.

The end portions 201, 202 including the fastening parts 203, 204 are formed of a second material, in particular a second biocompatible, non-resorbable material having a second tensile modulus. At contact surface 211 the two material types are connected to each other. Further, the end portions 201, 202 each have a stub 207, 208 that extends into the intermediate section 203.

Fig. 5 schematically shows different examples of possible geometries of the interface surface by which the end portion 501 is connected to the intermediate section 502. Fig. 5A shows a planar interface surface 503a. Figs. 5B through 5G show interface surfaces with differently shaped protrusions of the end portion into the intermediate section. Fig. 5B shows a donut shaped interface 503b. Fig. 5C shows an adjusted donut shape surface 503c, which has multiple grooves to enlarge the contact surface in multiple directions between the end portion 501 and the intermediate section 502. Fig. 5D shows an interface 503d having the shape of a block. Fig. 5E shows another interface 503e having the shape of a block, wherein the block of interface 503e is longer than the block of interface 503d. Fig. 5F shows an interface 503f with a wedge. Fig. 5G shows an interface 503g with a long wedge, wherein the wedge of interface 503f is longer than the wedge of interface 503g. In alternative embodiments, the intermediate section 502 may have a protrusion into the end portion 501 instead of or in addition to a protrusion of the end portion 501 into the intermediate section 502. That protrusion may have a similar shape as the shown shapes.

In cross section, as indicated at numeral 209, the intermediate section may have a wedge shape, wherein the intermediate section may have a larger thickness at its outer circumference 215 compared to its inner circumference, as illustrated in Fig. 3.

The more flexible the implant, the less force is needed to push the implant out of the joint. Thereby the forces in circumferential direction (see arrow 210) are reduced. Preferably, the flexible implant is still stiff enough to keep its function as load sharing structure.

Flexibility may also improve the contact area between the implant and the cartilage. The shape of the part of the condyle, which is in contact with the meniscus, may change during walking. Due to the increased flexibility of the implant, the shape of the implant may better follow the change in shape of the condyle during a walking motion. The contact area between condyle, cartilage and implant may adapt to motion.

The present disclosure may provide a durable implant with a long survival in the knee without tearing. To prevent tears in the implant one can increase the strength by choosing a polymer with higher strength in circumferential direction (arrow 210) but due to restrictions in polymer mechanical material characteristics this leads automatically to an increased tensile modulus and higher stress on the materials and a potentially unacceptable tensile modulus in all other planes which makes it less compliant to different knee sizes and forms.

This tensile modulus may be lower than that of the native meniscus in circumferential direction.

In certain embodiments of the meniscus implant, in particular its middle section has a certain flexibility. However, preferably the implant is not too flexible, especially not with regard to the horizontal plane (the plane of the tibial plateau). This is because the implant should not flip over after a deep bending of the joint. In a native meniscus, this also occurs when the meniscus tears away from the capsule. This fixation to the capsule keeps the meniscus in place. When the native meniscus tears away from the posterior capsule, the meniscus as a whole can fold forward in the knee and become nonfunctional and the knee can become painful. This is called having a bucket handle tear. This condition can occur because the native meniscus is very flexible in the horizontal plane and relies on fixation to the capsule. The native meniscus is fixed to the capsule at several places, not only at the horns. By making the implant stiff enough in the horizontal plane (the plane of the tibial plateau), the two-point fixation at the ends is sufficient to prevent such a 'flip over'. Thus, the implant does not need sutures to the peripheral capsule of the knee.

In certain embodiments, the stiffer end part extends into the flexible middle section of the implant (see the example of the stubs 207, 208). In certain embodiments, the implant may be manufactured using injection molding. During the injection molding process, the end parts may be molded first in respective first molds, and cooled down. After that, the end parts may be placed in a second mold and hot liquid flexible polymer may be injected against the stiffer polymer. The stubs of the stiffer end part that extend in to the flexible middle section provide a relatively large contact area, which ensures a good bond between each end part and the middle section.

Moreover, the 'stub' extension of the stiffer polymer end parts into the middle section works as a 'tear stopper': under high loads, the transition part of the stiffer to the more flexible part could remain a weak spot susceptible of fatigue tears. In case the connection between the body and the end part starts to fail, a tear may be created, but the tear will be stopped by the stiffer material stub.

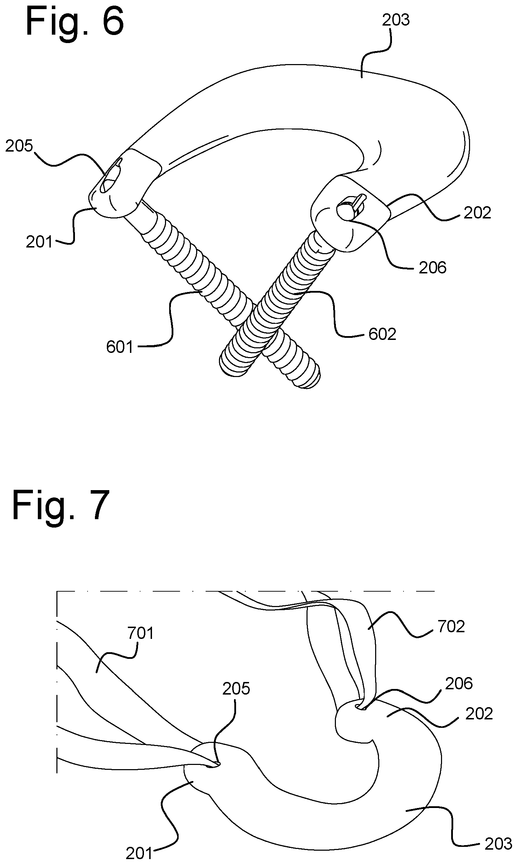

Fig. 6 shows a meniscus prosthesis according to an embodiment with middle section 203 and first and second ends 201, 202. The ends 201, 202 have through holes 205, 206 as the fastening parts. The figure further shows screws 601, 602 extending through the through holes 205, 206. These screws may be used to attach the prosthesis to the bone, e.g. at the location of the horns.

Fig. 7 shows a meniscus prosthesis according to an embodiment with middle section 203 and first and second ends 201, 202. The ends 201, 202 have through holes 205, 206 as the fastening parts. The figure further shows tapes 601, 602 extending through the through holes 205, 206, respectively. These tapes may be used to attach the prosthesis to the bone, e.g. at the location of the horns. For example, a through hole may be prepared in the bone so the tape can be fixed to the bone by allowing the tape to extend through the through hole 205 or 206 and through the through hole in the bone, and the ends of the tape may be fixed together with a knot, for example.

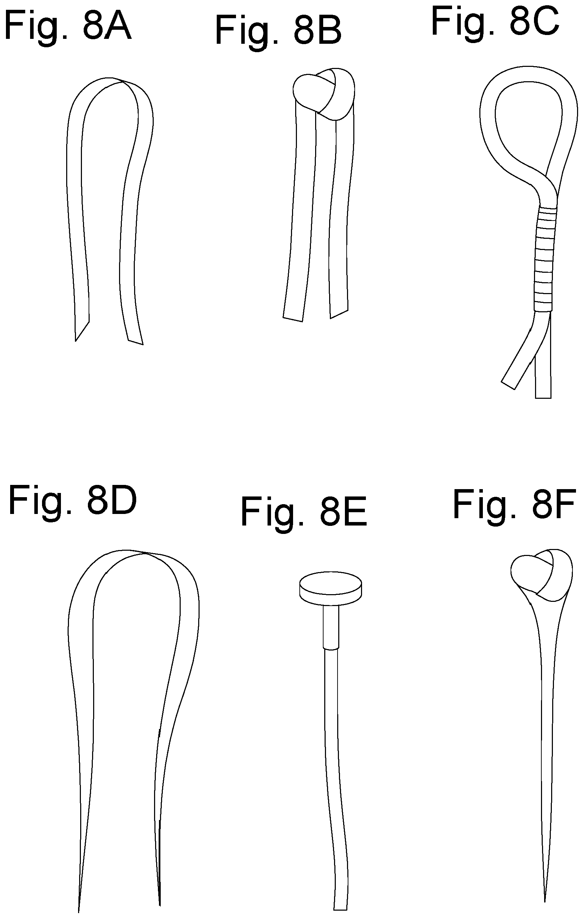

Fig. 8 shows several aspects of tape fixation. Fig. 8A shows a tape, similar to tapes 701 and 702. In this configuration, the tape goes through the hole 203 or 204 and through the corresponding drill hole in the tibia bone.

Fig. 8B shows a tape with a knot. In this configuration the two end parts of the tape go through the same hole 203 or 204 and the knot prevents the tape from slipping through the hole and lies on the implant horn.

Fig. 8C shows a tape with a loophole. The loophole may extend through the through hole 205 or 206 and through the through hole in the bone, so that the end is in effect attached to the bone.

Fig. 8D shows a tape with tapered ends. The tape is pulled into the drill hole and the tighter the tape is fixed in the drill holes, the less wear of the tape occurs against the bone. The tapered ends of the tape allow a smaller diameter of the drill hole. That is, it is easier to pull a tape with a tapered end into a drill hole.

Fig. 8E shows a tape member in form of a braking cable. The block at the end of the braking cable may not fit through the through hole 205, 206, so that with the tape extending through the through hole 205, 206 of the prosthesis and through the through hole in the bone the prosthesis is attached to the bone. Fig. 8F shows a tape with a knot on one end and tapered on the other end. The knot works similar to the block at the end of a braking cable. This configuration combines the advantages of the two configurations of Figs. 8D and 8E.

In the configurations of Figs. 7 and 8, the tape can be replaced with another flexible fastening member, such as a wire, a cable, or a strip.

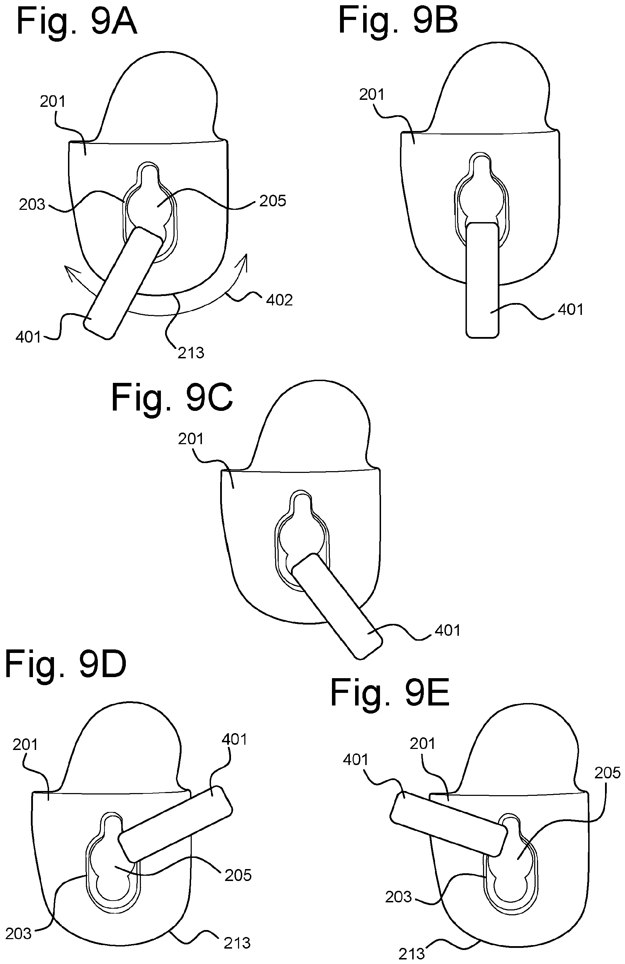

A tape fixation of the implant through a hole in the end portion allows more motion of the implant and the round shape of the horn allows easier movement. In case the implant is fixated to the bone with a tape, the round shape of the end part enables the tape to move around the end part during knee joint motion. This is illustrated in Fig. 9A to 9C. The part of the edge 213, 214 of the end portion 201, 202 facing away from the intermediate section 203 is rounded, so that the tape 401 can rotate with respect to the meniscus prosthesis along the direction of arrow 402. Specifically, the edge 213 is rounded around the fastening means 203, 204 and through hole 205, 206. As shown in Fig. 9A to 9C, the tape 401 can then more easily move between different positions along the edge 213, 214.

Also, in certain embodiments, as shown in Fig. 9D, the through hole 205 is elongated in the longitudinal direction of the meniscus prosthesis, allowing the tape to move back and forth inside the through hole, from e.g. positions as shown in Figs. 9A to 9C to a position as shown in Fig. 9D or Fig. 9E. This provides the meniscus prosthesis with more freedom to migrate through the joint.

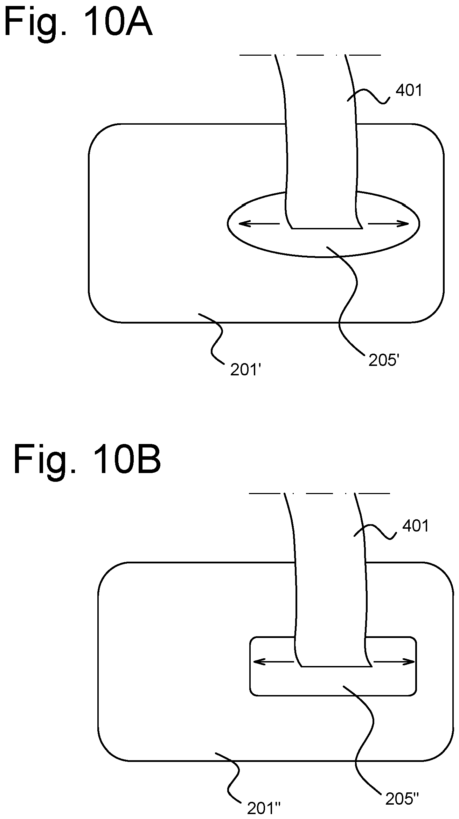

Fig. 10A shows another example of an elongated through hole 205' of an end portion 201'. The end portion 201' is sketched diagrammatically. The figure shows an elliptical through hole 205'. The tape 401 can move back and forth along the longest diameter of the through hole 205'. Fig. 10B shows yet another example of an elongated through hole 205" of an end portion 201". The end portion 201" is sketched diagrammatically. The through hole 205" is rectangular.

In the case of an implant that is allowed a defined, limited, movement in the joint, its attachments and the meniscus body should preferably be flexible. However, connecting a tensile stiff structure, like tape or suture band, directly to a flexible implant body may lead to an abrupt difference in tensile modulus and/or tensile strength, which may lead to creep or wear in the flexible part. A more gradual transition from the stiff part to the flexible part, provided by the stiffer end portions of certain embodiments, may reduce chance of failure.

In certain embodiments, the meniscus prosthesis has a C-shaped prosthesis body. This body is not reinforced, is flexible, and is able to stretch to withstand the all the loads from the femur condyle. On both ends the prosthesis body is connected with a structure with a hole through it (located where the implant is fixed to the tibial plateau). This structure is more rigid than the body and the purpose of the hole is to hold a flexible connector (e.g. a cord, tape, or suture) that is fixed to the tibia. In certain alternative embodiments, the solid structure is fixed to the tibia with a screw or pin through the hole.

In certain embodiments, two flexible cords, tapes, or sutures, or pins or screws, are attached to the tibia and fixed to a hole through a rigid, solid interface (with little to no flexibility or stretch). For example, an elastic modulus of the solid interface (i.e. the end part) may be at least 100 MPa. Both rigid interfaces are connected to each other by a flexible (not reinforced) prosthesis body that can elongate up to 10-20% under normal walking conditions. For example, an elastic modulus of the prosthesis body (i.e. the middle section) may be greater than 5 MPa and less than 100MPa, preferably less than 50 MPa. This prosthesis body has a C-shape and the rigid interfaces are attached to the ends of the C-shape.

The prosthesis disclosed herein is not limited to a meniscus prosthesis. The features disclosed herein in respect of a meniscus prosthesis may be applied to other types of prosthesis. In general, an arc-shaped prosthesis may comprise a first end 201 having a first fastening part 203 for securing the first end 201 to a bone surface; a second end 202 having a second fastening part 204 for securing the second end 202 to a bone surface; and a curved intermediate section 203 connecting the first end 201 and the second end 202, wherein the curved intermediate section 203 is made of a single first biocompatible, non-resorbable material that has a tensile modulus of at most 160 MPa as determined by ISO 527-1. This prosthesis may be further defined as disclosed herein in respect of the meniscus prosthesis. For example, a shoulder labrum prosthesis may be made having features similar to the features of the meniscus prosthesis, for example to replace and/or reinforce the shoulder labrum.

The examples and embodiments described herein serve to illustrate rather than limit the invention. The person skilled in the art will be able to design alternative embodiments without departing from the spirit and scope of the present disclosure, as defined by the appended claims and their equivalents. Reference signs placed in parentheses in the claims shall not be interpreted to limit the scope of the claims. Items described as separate entities in the claims or the description may be implemented as a single hardware or software item combining the features of the items described.

## Claims

1. An arc-shaped meniscus prosthesis, comprising
a first end (201) having a first fastening part (203) for securing the first end (201) to a bone surface;
a second end (202) having a second fastening part (204) for securing the second end (202) to a bone surface;
a curved intermediate section (203) connecting the first end (201) and the second end (202),
wherein the curved intermediate section (203) is made of a single first biocompatible, non-resorbable material that has a tensile modulus of at most 160 MPa as determined by ISO 527-1.

2. The meniscus prosthesis of claim 1, wherein only the first end and the second end of the prosthesis have fastening parts.

3. The meniscus prosthesis of claim 1, wherein the first material is isotropic with regard to the tensile modulus and the curved intermediate section is a monolithic piece of the first material.

4. The meniscus prosthesis of any preceding claim, wherein the first material has a tensile modulus between 0.1 MPa and 160 MPa measured according to ISO 527-1, preferably between 5 MPa and 100 MPa measured according to ISO 527-1,preferably between 5 MPa and 15 MPa measured according to ISO 527-1.

5. The meniscus prosthesis of any preceding claim, wherein the meniscus prosthesis as a whole is stiff enough to, when secured to the bone surface at the first end and at the second end, prevent dislocation of the curved intermediate section.

6. The meniscus prosthesis of any preceding claim, wherein the first end comprises a second biocompatible, non-resorbable material that has a tensile modulus of at least 100 MPa as determined by ISO 527-1, and the second end comprises a third biocompatible, non-resorbable material that has a tensile modulus of at least 100 MPa as determined by ISO 527-1, wherein the second material and the third material can be the same material or different materials.

7. The meniscus prosthesis of claim 6, wherein the second material and the third material each have a tensile modulus between 100 MPa and 3500 MPa measured according to ISO 527-1, preferably between 100 MPa and 1000 MPa, more preferably between 100 MPa and 250 MPa, measured according to ISO-527-1.

8. The meniscus prosthesis of any one of claims 6 to 7, wherein the first end of the meniscus prosthesis is made entirely of the second material and the second end is made entirely of the third material.

9. The joint prosthesis of any one of claims 6 to 8, wherein
the first end comprises a first protrusion made of the second material, wherein the first protrusion engages with a corresponding recess in the middle section, and
the second end comprises a second protrusion made of the third material, wherein the second protrusion engages with a corresponding recess in the curved intermediate section.

10. The joint prosthesis of claim 6, wherein the first end and/or the second end comprises a core made of the second material and a cushioning material that covers the core.

11. The meniscus prosthesis of any preceding claim, wherein a transverse cross-sectional area of the curved intermediate section is wedge shaped, with a larger thickness at an outer circumference of the curved intermediate section than at an inner circumference of the curved intermediate section.

12. The meniscus prosthesis of any preceding claim, wherein the first material, the second material, and the third material each comprise a thermoplastic material, preferably a polyurethane, more preferably a polycarbonate urethane.

13. The meniscus prosthesis of any preceding claim, wherein
the first fastening part comprises a first through-hole configured to receive a connector for securing the first end to a bone surface; and
the second fastening part comprises a second through-hole configured to receive a connector for securing the second end to a bone surface.

14. A method of manufacturing an arc-shaped meniscus prosthesis, comprising
molding at least part of a first end of the arc-shaped meniscus prosthesis, the at least part of the first end having a first fastening part for securing the first end to a bone surface;
molding at least part of a second end of the arc-shaped meniscus prosthesis, the at least part of the second end having a second fastening part for securing the second end to a bone surface; and
molding a curved intermediate section connecting the first end and the second end,
wherein the curved intermediate section is made of a first biocompatible, non-resorbable material that has a tensile modulus of at most 160 MPa as determined by ISO 527-1.

15. The method of claim 13, wherein the at least part of the first end is made of a second biocompatible, non-resorbable material and the at least part of the second end is made of a third biocompatible, non-resorbable material, wherein the second material and the third material each have a tensile modulus of at least 100 MPa, preferably between 100 MPa and 1000 MPa, more preferably between 100 MPa and 250 MPa, measured according to ISO-527-1, wherein the second material and the third material can be the same material or different materials.
